(19) Europäisches Patentamt
European Patent Office
Office européen des brevets

(11) Publication number: **0 068 557**
**B1**

# (12) EUROPEAN PATENT SPECIFICATION

(45) Date of publication of patent specification: **09.12.87**

(21) Application number: **82200735.7**

(22) Date of filing: **14.06.82**

(51) Int. Cl.[4]: **C 07 D 515/04,**
C 07 D 517/04,
C 07 D 515/06,
C 07 D 517/06,
C 07 D 515/16,
C 07 D 517/16, A 01 N 43/90
// (C07D515/04, 291:00,
285:00),(C07D517/04, 293:00,
293:00),(C07D515/06, 291:00,
291:00),(C07D515/06, 291:00,
285:00),(C07D517/06, 293:00,
293:00),(C07D515/16, 335:00,
291:00, 291:00)

## (54) Herbicidal compositions containing heterocyclic pentalenes.

(30) Priority: **24.06.81 GB 8119403**

(43) Date of publication of application:
**05.01.83 Bulletin 83/01**

(45) Publication of the grant of the patent:
**09.12.87 Bulletin 87/50**

(84) Designated Contracting States:
**AT BE CH DE FR GB IT LI NL SE**

(56) References cited:
**EP-A-0 003 261**

**Bull. Soc. Chim. France, 1979, II-200 to II-207**

**Bull. Soc. Chim. France, 1971, p. 4591**

**Tetrahedron Letters, no. 18, 1972, p. 1836**

**Burger Chem., no. 89, 1977, p. 420**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Clark, Michael Thomas**
**1 Hanover Close**
**Sittingbourne Kent (GB)**
Inventor: **Gilmore, Ian James**
**32 Waterloo Road**
**Sittingbourne Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 557 B1

**Description**

The present invention relates to a composition for and a method of controlling undesired plant growth and to compounds for use in such a composition or method.

Certain oxadithiadiaza- and dioxathiadiaza-2,5-pentalenes having the following fused ring structure

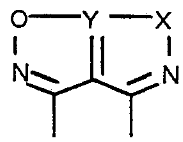

wherein Y is sulphur and X is sulphur or oxygen are known from Perrier and Vialle, *Bull. Soc. Chim. France,* 1979, II—199—208 and Beer and Poole, *Tetrahedron Letters* No. 18, 1972, 1835-36. The references teach that the compound having this fused ring structure can be prepared by reacting a dioxime of a B-diketone with sulphur monochloride or sulphur dichloride, the product typically being a mixture of the two ring systems. Certain dioxaselenadiaza- and dioxatelluradiaza-2,5-pentalenes wherein Y is selenium and tellurium and X is oxygen are also known from Perrier and Vialle, *Bull. Soc. Chim. France,* 1971 No. 12, 4591—2. None of the references teach that the compounds described have herbicidal activity or any other useful biological property.

It has now been found that certain oxadithiadiaza- dioxaselenadiaza-, dioxatelluradiaza- and dioxathiadiaza-2,5-pentalenes have useful herbicidal properties.

The invention provides a herbicidal composition which comprises at least two carriers, at least one of which is a surface-active agent, together with, as active ingredient, a compound of the general formula I or where possible an acid addition salt thereof:

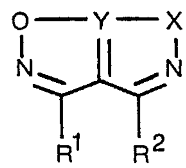

(I)

in which Y represents a sulphur, selenium or tellurium atom; $R^1$ and $R^2$ each independently represent an alkyl group of up to 6 carbon atoms, a phenyl group or a group

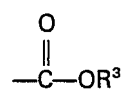

wherein $R^3$ is an alkyl group of up to 6 carbon atoms; or $R^1$ and $R^2$ together represent an alkylene linkage containing up to 4 carbon atoms in the chain or an alkylene linkage containing up to 3 carbon atoms and one heteroatom selected from sulphur, nitrogen and oxygen in the chain, each linkage being optionally substituted with up to 3 alkyl groups each containing up to 12 carbon atoms, up to 3 phenyl groups, a benzyl group, an acyl group of up to 12 carbon atoms, a pyridyl group or salt thereof, an alkoxy substituted phenyl group containing up to 6 carbon atoms in the alkyl portion of the alkoxy moiety or a furyl group, and when the hetero atom is sulphur the oxidised derivatives thereof; and X represents an oxygen atom or, when Y represents a sulphur atom, X may alternatively represent a sulphur atom.

A preferred group of compounds which are useful in the compositions of the invention are those of general formula II or where possible an acid addition salt thereof:

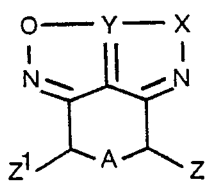

(II)

in which X and Y have the meaning given in general formula I, Z represents a hydrogen atom, an alkyl group of up to 12 carbon atoms, a phenyl group, a pyridyl group or salt thereof or a benzyl group; $Z^1$ represents a hydrogen atom, an alkyl group of up to 6 carbon atoms a pyridyl group or salt thereof or a phenyl group and A represents a group

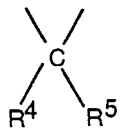 , 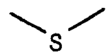 , 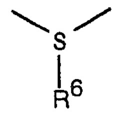 , 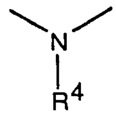 , 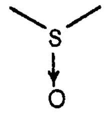 , 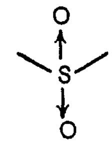 or 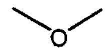

wherein $R^4$ represents a hydrogen atom, an alkyl group of up to 12 carbon atoms, a benzyl group or a pyridyl group or salt thereof, or a group

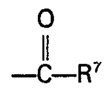

wherein $R^7$ is an alkyl or alkenyl group of up to 12 carbon atoms, an aryl group of up to 12 carbon atoms, an aralkyl group of up to 9 carbon atoms or an alkaryl group of up to 9 carbon atoms, $R^5$ represents a hydrogen atom or an alkyl group of up to 6 carbon atoms and $R^6$ represents an alkyl group of up to 12 carbon atoms or a benzyl group, with the proviso that both of Z and $Z^1$ can not simultaneously be a pyridyul group or a salt thereof.

Most preferred compounds for use in the herbicidal compositions of the invention are those compounds according to general formula II in which Z and $Z^1$ each represent a hydrogen atom or an alkyl group of up to 3 carbon atoms and A is a group

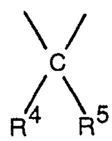 , 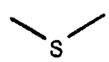 or 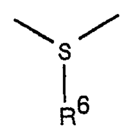

wherein $R^4$ is a hydrogen atom or an alkyl group of up to 9 carbon atoms, especially methyl, $R^5$ is a hydrogen atom or an alkyl group or up to 3 carbon atoms, especially methyl and $R^6$ is an alkyl group of up to 9 carbon atoms, especially methyl.

Preferably, Y represents a sulphur atom.

Many of the compounds of the general formula I are novel and the invention therefore also provides these novel compounds *per se.* The novel compounds are those compounds and salts thereof as defined above in general formula I wherein Y is sulphur, provided that one of $R^1$ and $R^2$ can not be methyl when the other is ethoxycarbonyl and further provided that when $R^1$ and $R^2$ together represent an alkylene linkage of 3 or 4 carbon atoms, the carbon chain of the linkage must be substituted with at least one of the groups indicated as optional substitutents and when the alkylene linkage contains 3 carbon atoms the middle carbon atom must not be substituted with two methyl groups if the other two carbon atoms are unsubstituted. Preferred novel compounds of the present invention are those compounds according to general formula II above wherein Y is sulphur with the proviso that both of Z and $Z^1$ can not simultaneously be hydrogen when A represents a group

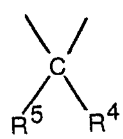

and $R^4$ and $R^5$ are the same, each representing hydrogen or methyl.

The invention also provides a process for the preparation of a novel compound of the general formula I which comprises reacting a dioxime of the general formula

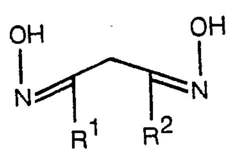 (III)

with sulphur monochloride and/or sulphur dichloride. The product of this reaction is typically a mixture of the desired oxadithiadiaza-2,5-pentalene of general formula I (the compound wherein X and Y are sulphur atoms) and the desired dioxathiadiaza-2,5-pentalene of general formula I (the compound wherein X is an oxygen atom and Y is a sulphur atom). The ratio in which these two products form during the reaction is

rather variable depending on the chemical make up of the dioxime starting material used and the type of sulphur chloride employed. Typically when sulphur dichloride is used in preference to sulphur monochloride, the yeild of dioxathiadiaza-2,5-pentalene is enhanced. The reaction is suitably conducted in a polar organic solvent such as tetrahydrofuran or diethyl ether which is inert under the reaction conditions employed. The reaction temperature will typically range between −80 and −20°C. When the reaction is carried out in batch fashion it is desirable to slowly add the sulphur chloride reactant to the dioxime in polar organic solvent at a temperature of from −80 to −20°C, to maintain this temperature for a time interval, for example 8 to 12 hours, and then to allow the reaction mixture to warm slowly to ambient temperature (20°C), holding it at this temperature for an additional 6 to 16 hours. Generally the total reaction time will range between 16 and 30 hours.

As noted previously, the reaction product of the reaction between the dioxime of general formula III and a sulphur chloride generally contains a mixture of the two compounds according to general formula I which are identical in structure except for the nature of the X substituent, X being a sulphur atom or an oxygen atom. The desired oxadithiadiaza-2,5-pentalene component and the dioxathiadiaza-2,5-pentalene component are suitably separated from the mixed reaction product using conventional techniques. In a preferred separtion process, the two components in the reaction product are isolated by chromatography on silica using an eluant such as petroleum/ether or methylene chloride. After isolation, the components can be further purified using conventional solvent recrystallization techniques, alcohols such as ethanol and hydrocarbon solvents such as hexane, cyclohexane and benzene being suitable recrystallization solvents.

The dioximes of general formula III are generally known compounds, being prepared by reaction of the appropriate betadiketone of the formula

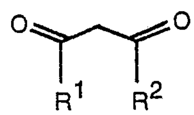

(IV)

with hydroxylamine. This dioxime forming reaction is typically carried out by reacting stoichiometric amounts of the diketone (1 mole) and hydroxylamine (2 moles) at temperature of from 25 to 100°C for 10 to 60 minutes in a polar solvent.

The precursor B-diketones (IV) are also known compounds which may be obtained by a variety of conventional synthetic techniques. For example, the diketones of formula IV in which $R^1$ and $R^2$ taken together represent an optionally substituted alkylene linkage are suitably prepared by condensation of the appropriate ester, diester or ketoester with a methyl alkenyl ketone followed by cyclization of the resulting saturated keto ester. Alternatively, the carbon-carbon double bond may be in the ester reactant and a methyl alkyl ketone may be employed. These condensations and ring closure reactions are typically carried out in the presence of a base such as sodium ethoxide or sodium hydride. Diketones of formula IV wherein $R^1$ and $R^2$ together represent an optionally substituted alkylene linkage containing a heteroatom in the chain are conveniently prepared by reacting an ester containing an amino, alcohol or mercapto functional group with a halogenated methyl ketone followed by cyclization of the resulting keto ester; the first reaction being carried out in the presence of an acid acceptor such as sodium bicarbonate or sodium methoxide and the second reaction being conducted in the presence of a base such as sodium hydride in ethanol or potassium tert butoxide in tert. butanol.

Compounds of the general formula I wherein X is oxygen and Y is selenium or tellurium may be prepared by methods analogous to those described in *J. Chem. Soc.* 274 (1949) King and Felton and *Bull. Chim. Soc. Fr.* 4517 (1970) 4591—21 (1971) Perrier and Vialle.

The compounds of the general formula I have useful herbicidal properties and the invention provides a method for combating undesired plant growth at a locus, which comprises applying to the locus a compound or a composition according to the invention. For optimum herbicidal effect, the compounds or compositions of the invention are applied at an active compound dosage from 0.02 to 10 kg/ha, preferably 0.1 to 5 kg/ha.

A carrier in a composition according to the invention is any material with which the active ingredient is formulated to facilitate application to the locus to be treated, which may for example be a plant seed or soil, or to facilitate storage, transport or handling. A carrier may be a solid or a liquid, including a material which is normally gaseous but which has been compressed to form a liquid, and any of the carriers normally used in formulating herbicidal compositions may be used.

Suitable solid carriers include natural and synthetic clays and silicates, for example natural silicas such as diatomaceous earths; magnesium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for

example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated caster oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. In general, such compositions may contain from 0.5 to 95% w of the active ingredient with the remainder being adjuvants conventionally employed in such compositions, that is carriers and/or surface active agents. Wettable powders usually contain 25, 50 and 75% w of active ingredient and usually contain in addition to solid inert carrier, 3—10% w of a dispersing agent and, where necessary, 0—10% w of stabiliser(s) and/or other additives such as penetrants of stickers. Dusts are usually formulated as a dust concentrate having a similar compositions to that of a wettable powder but without a dispersant, and are diluted in the field with further solid carrier to give a composition usually containing $\frac{1}{2}$—10% w of active ingredient. Granules are usually prepared to have a size between 10 and 100 BS mesh (1.676—152 mm), and may be manufactured by agglomeration or impregnation techniques. Generally, granules will contain $\frac{1}{2}$—25% w active ingredient and 0—10% w of additives such as stibilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thisotropic agents, 0—10% w of other additives such as defoamers, corrosion inhibitors, stabilisers, penetrants and stickers, and water or an organic liquid in which the active ingredient is substantially organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and have a thick 'mayonnaise'-like consistency.

The composition of the invention may also contain other ingredients, for example, other compounds possessing herbicidal, insecticidal or fungicidal properties.

The following Examples illustrate the invention.

Example I

Preparation of a) 7,8-dihydro-6H-(1,2,5)oxathiazolo(4,3,2-hi)-(2,1,3)benzoxathiazole-3-$S^{1V}$

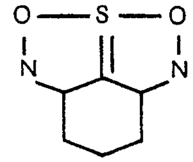

and b/ 7,8-dihydro-6H-(1,2,3)dithiazolo(4,5,1-hi)(2,1,3)benzoxathiazole-3-$S^{1V}$

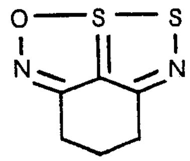

1.3-Cyclohexane dioxime (20.0 g, 140.8 mmol) was suspended in dry tetrahydrofuran (750 ml) and the temperature of the mixture was lowered to −70°C with stirring in an acetone-dry ice bath. Sulphur monochloride (39.0 g, 289 mmol) was added dropwise with stirring and the temperature was maintained at −70°C for an additional 12 hours. The reaction mixture was then allowed to warm to room temperature and it was poured into water. The aqueous mixture was extracted with chloroform and the chlorform extract was dried over sodium sulphate. The dried mixture was then chromatographed over silica, eluting with chloroform. The first fraction made up of sulphur was discarded. A second orange fraction was collected and the orange residue was recrystallized from cyclohexane to afford 10.6 g of orange needles, corresponding to a 44.4% yield of compound a) 7,8-dihydro-6H-(1,2,5)-oxathiazolo(4,3,2-hi)(2,1,3)-benzoxathiazole-3-$S^{1V}$, melting at 66—67°C.

| *Elemental Analysis* | C | H | N |
|---|---|---|---|
| Calculated for $C_6H_6N_2O_2S$ | 42.4 | 3.5 | 16.5 |
| Found | 42.2 | 3.6 | 16.3 |

a third brown fraction was also collected and the black/brown residue was recrystallized from cyclohexane to afford 3.5 g of brown needles corresponding to a 13.4% yield of compound b) 7,8-dihydro-6H-(1,2,3)-dithiazolo(4,5,1-hi)(2,1,3)benzoxathiazole-3-$S^{1V}$, melting at 154—155°C.

| *Elemental Analysis* | C | H | N |
|---|---|---|---|
| Calculated for $C_6H_6N_2OS_2$ | 38.7 | 3.2 | 15.1 |
| Found | 39.0 | 3.1 | 15.0 |

Example 2

Alternative preparation of a) 7,8-dihydro-6H-(1,2,5)oxathiazolo(4,3,2-hi)(2,13)benzoxathiazole-3-$S^{1V}$ and b) 7,8-dihydro-6H-(1,2,3)dithiazolo(4,5,1-hi)(2,1,3)benzoxathiazole-3-$S^{1V}$

1.3-Cyclohexanone dioxime (5.4 g, 38 mmol) was suspended in sodium-dried tetrahydrofuran and the temperature of the mixture was lowered to −70°C in an acetone-dry ice bath. The mixture was stirred vigorously and sulphur dichloride (8.0 g 77 mmol) was added dropwise while the temperature was maintained at −70°C. The mixture was then stirred for an additional 8 hours at −70°C and then allowed to warm at room temperature with continued stirring for 24 hours. The reaction mixture was then poured into cold water (500 ml) and extracted with hot toluene. The extract was dried over sodium sulphate, filtered and the solvent removed by distillation under reduced pressure. The residue was chromatographed over silica gel, elution with chloroform afforded an orange-yellow solid followed by a second fraction of a dark orange solid which were both recrystallized from cyclohexane to afford, respectively 1.6 g of compound a) corresponding to a 25% yeild, m.p. 65—66°C and 0.1 g of compound b) corresponding to a 2% yield, m.p. 158—159·C.

| *Elemental Analysis* | C | H | N |
|---|---|---|---|
| *Compound a)* | | | |
| Calculated for $C_6H_6N_2O_2S$ | 42.4 | 3.5 | 16.5 |
| Found | 42.3 | 3.6 | 16.2 |
| *Compound b)* | | | |
| Calculated for $C_6H_6N_2OS_2$ | 38.7 | 3.2 | 15.1 |
| Found | 38.2 | 3.2 | 14.6 |

Example 3

Preparation of a) 7,8-dihydro-7,7-dimethyl-6H-(1,2,5)oxathiazolo-(4,3,2-hi)(2,1,3)benzoxathiazole-3-$S^{1V}$

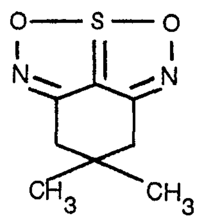

and b) 7,8-dihydro-6,7-dimethyl-6H-(1,2,3)dithiazolo(4,5,1-hi)(2,1,3)benzoxathiazole-3-$S^{1V}$

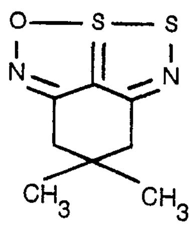

Dimedone dioxime (25.5 g, 150 mmol) was suspended in dry tetrahydrofuran and maintained at −70° to −80°C in an acetone-dry ice bath with stirring. Subsequently, sulphur monochloride was added dropwise to the stirred mixture and it was held for an additional 12 hours at −70° to −80°C with stirring. The reaction mixture was then allowed to warm to room temperature and held for an additional 48 hours. The reaction mixture was then poured into water (1500 ml) and extracted with hot toluene. The toluene extract was filtered with glass wool to remove any sulfur-containing solids, dried over sodium sulphate and the toluene solvent was removed under vacuum. The residue was chromatographed on silica first with toluene to obtain an orange solid and then with chloroform to obtain a black/orange solid. The orange solid was recrystallized from hexane to afford 8.4 g of orange platelets, corresponding to a 28.4% yield of 7,8-dihydro-7,7-dimethyl-6H-(1,2,5)oxathiazolo(4,3,2-hi)(2,1,3)benzoxathiazole-3-$S^{1V}$, m.p. 65.5°C.

| *Elemental Analysis* | C | H | N |
|---|---|---|---|
| Calculated for $C_8H_{10}N_2O_2S$ | 48.5 | 5.1 | 14.1 |
| Found | 48.5 | 5.0 | 14.0 |

The black/orange solid was recrystallized from cyclohexane to afford 8.2 g of black irridecent needles, corresponding to a 25.6% yield of 7,8-dihydro-7,7-dimethyl-6H-(1,2,3)dithiazolo(4,5,1-hi)(2,1,3)benzoxathiazole-3-$S^{1V}$, m.p. 97°C.

| *Elemental Analysis* | C | H | N |
|---|---|---|---|
| Calculated for $C_8H_{10}N_2OS_2$ | 44.9 | 4.7 | 13.1 |
| Found | 44.7 | 4.5 | 12.8 |

Example 4

Alternative preparation of a) 7,8-dihydro-7,7-dimethyl-6H-(1,2,5)-oxathiazolo(4,3,2-hi(2,13)benzoxathiazole-3-$S^{1V}$ and b) 7,8-dihydro-7,7-dimethyl-6H-(1,2,3)dithiazolo(4,5,1-hi)(2,1,3)benzoxathiazole-3-$S^{1V}$

Dimedone dioxime (8.5 g, 50 mmol) was suspended in dry tetrahydrofuran (200 ml) at −60°C in an acetone-dry ice bath and sulphur dichloride (5.65 g, 55 mmol) was added dropwise with vigorous stirring to give a yellowish-orange solution. This solution was stirred at −60 to −70°C for 8 hours after which it was warmed to room temperature and held for an additional 36 hours with stirring. The solution was poured into water and extracted with toluene by refluxing portions of the aqueous mixtures with toluene. The toluene extracts were combined and dried over magnesium sulphate. The toluene was removed by evaporation in a vacuum and the residue was chromatographed on silica gel eluting first with toluene and then with chloroform. The first fraction eluted with toluene afforded an orange solid which was recrystallized from hexane to give 1.7 g of compound a) as orange needles, corresponding to a 17% yield, m.p. 64°—65°C. The second fraction eluted with chloroform afforded a dark orange/black solid which was recrystallized from cyclohexane to give 0.6 g of black crystals corresponding to a 5.7% yield of compound b) m.p. 98°—100°C.

| *Elemental Analysis* | C | H | N |
|---|---|---|---|
| *Compound d)* | | | |
| Calculated for $C_8H_{10}N_2O_2S$ | 48.5 | 5.1 | 14.1 |
| Found | 48.5 | 5.0 | 14.0 |
| *Compound b)* | | | |
| Calculated for $C_8H_{10}N_2OS_2$ | 44.9 | 4.7 | 13.1 |
| Found | 44.7 | 4.5 | 12.8 |

Example 5

Preparation of a) 7,8-dihydro-6,8-dimethyl-6H-(1,2,5)oxathiazolo-(4,3,2-hi)(2,1,3)benzoxathiazole-3-$S^{1V}$

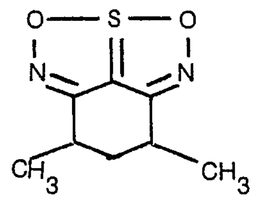

and b) 7,8-dihydro-6,8-dimethyl-6H-(1,2,3)dithiazolo(4,5,1-hi)(2,1,3)benzoxathiazole-3-$S^{1V}$

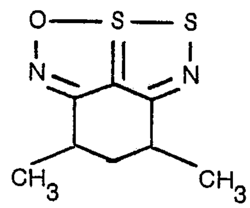

4,6-Dimethyl-1,3-cyclohexanone dioxime (11.9 g, 70 mmol) was suspended in dry tetrahydrofuran (350 ml) and cooled in an acetone-dry ice bath to −70°C, at which point sulphur monochloride (20.25 g, 150 mmol) was added dropwise with stirring. The reaction mixture was then stirred vigorously at −70°C for an additional 12 hours afterwhich it was allowed to warm to room temperature and held for 24 hours. The reaction mixture was then poured into water and extracted with hot toluene. The toluene extract was dried with magnesium sulphate and the solvent was removed by evaporation in a vacuum. The residue was chromatographed on silica, eluting with chloroform. The first fraction made up of sulphur was discarded. The second fraction, pale yellow in colour, was collected and recrystallized from petroleum ether (b.p. 40—60°C) to afford 0.6 g of pale orange needles, corresponding to a 4.4% yeild of 7,8-dihydro-6,8-dimethyl-6H-(1,2,5)oxathiazolo(4,3,2-hi)(2,1,3)benzoxathiazole-3-$S^{1V}$, m.p. 92—93°C.

| *Elemental Analysis* | C | H | N |
|---|---|---|---|
| Calculated for $C_8H_{10}N_2O_2S$ | 48.5 | 5.1 | 14.1 |
| Found | 48.3 | 5.0 | 14.1 |

The third fraction, deep orange in colour, was collected and recrystallized from cyclohexane to afford 10.9 g of dark brown orange needles, corresponding to a 73% yield of 7,8-dihydro-6,8-dimethyl-6H-(1,2,3)-dithiazolo(4,51-hi)(2,1,3)benzoxathiazole-3-$S^{1V}$, m.p. 113°—114°C.

| *Elemental Analysis* | C | H | N |
|---|---|---|---|
| Calculated for $C_8H_{10}N_2OS_2$ | 44.9 | 4.7 | 13.1 |
| Found | 44.6 | 4.9 | 12.7 |

Example 6

Alternative preparation of a) 7,8-dihydro-6,8-dimethyl-6H-(1,2,5)-oxathiazolo(4,3,2-hi)(2,1,3)benzoxathiazole-3-$S^{1V}$ and b) 7,8-dihydro-6,8-dimethyl-6H-(1,2,3)dithaizolo(4,5)1-hi(2,1,3)benzoxathiazole-3-$S^{1V}$

4,6-Dimethyl-1,3-cyclohexanone dioxime (13.6 g, 80 mmol was suspended in dry tetrahydrofuran and the mixture was cooled to −65°C in an acetone-dry ice bath with stirring. Sulphur dichloride (17.9 g, 174 mmol) was added dropwise to the cooled mixture and the resulting solution was stirred for 18 hours at −65 to −70°C. The reaction mixture was allowed to warm to room temperature and poured into water. The aqueous mixture was extracted with chloroform, the extract dried over magnesium sulphate and chromatographed over silica, eluting with chloroform. The first fraction consisting essentially of sulphur was discarded and the second and third fractions being yellow and brown/black, respectively, in colour were collected and recrystallized from ethanol. The second fraction afforded 7.1 g of compound a), corresponding to a 44.9% yield, m.p. 97°—99°C, while the third fraction gave 1.7 g of compound b), corresponding to a 9.9% yield, m.p. 110—112.5°C.

| *Elemental Analysis* | C | H | N |
|---|---|---|---|
| *Compound a)* | | | |
| Calculated for $C_8H_{10}N_2O_2S$ | 48.5 | 5.1 | 14.1 |
| Found | 48.5 | 5.1 | 14.2 |
| *Compound b)* | | | |
| Calculated for $C_8H_{10}N_2OS_2$ | 44.9 | 4.7 | 13.1 |
| Found | 44.2 | 4.6 | 12.8 |

Example 7

Preparation of a) 7,8-dihydro-7-phenyl-6H-(1,2,5)oxathiazolo-(4,3,2-hi)(2,1,3)benzoxathiazole-3-$S^{IV}$

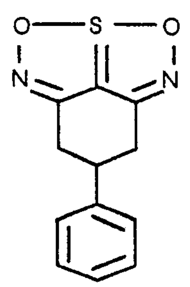

and b) 7,8-dihydro-7-phenyl-6H-(1,2,3)dithiazolo(4,5,1-hi)(2,1,3)-benzoxathiazole-3-$S^{IV}$

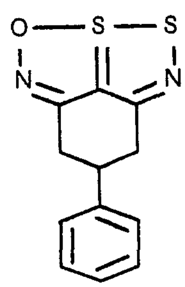

5-Phenyl-1,3-cyclohexanone dioxime (15.3 g, 70 mmol) was suspended in dry tetrahydrofuran (350 ml) and cooled in an acetone-dry ice bath to −70°C. Sulphur monochloride (20.25 g, 150 mmol) was added dropwise with stirring while the suspension was maintained at −70°C. The reaction mixture was held at −70°C for a further 12 hours and then allowed to warm to room temperature. The reaction mixture was then poured into water and extracted with hot toluene. The toluene extract was filtered through glass wool to remove the sulphur formed in the reaction and the filtered extract was dried over magnesium sulphate. The toluene solvent was removed by evaporation in a vacuum and the residue was chromatographed over silica, eluting with methylene chloride. The first fraction obtained, pale yellow in colour, was recrystallized from benzene to afford 4.7 g of pale yellow needles, corresponding to a 27.3% yeild of 7,8-dihydro-7-phenyl-6H-(1,2,5)oxathiazolo(4,3,2-hi)(2,1,3)benzoxathiazole-3-$S^{IV}$, m.p. 158—160°C.

| *Elemental Analysis* | C | H | N |
|---|---|---|---|
| Calculated for $C_{12}H_{10}N_2O_2S$ | 58.5 | 4.1 | 11.4 |
| Found | 58.5 | 4.1 | 11.4 |

The second fraction, deep orange in colour, was recrystallized from benzene to afford 3,4 g of black-brown needles, corresponding to a 18.5% yield of 7,8-dihydro-7-phenyl-6H-(1,2,3)dithiazolo-(4,5,1-

hi)(2,1,3)benzoxathiazole-3-$S^{IV}$, m.p. 168—170°C.

| *Elemental Analysis* | C | H | N |
|---|---|---|---|
| Calculated for $C_{12}H_{10}N_2OS_2$ | 55.0 | 3.8 | 10.7 |
| Found | 54.2 | 3.6 | 10.6 |

Examples 8—35

By methods analogous to those described in the foregoing Examples, the compounds 8—31 of formula I given in Table I were prepared. Compounds 32—35 of formula I given in Table I were prepared by methods analogous to those described in *J. Chem. Soc.* 274 (1949) King and Felton.

TABLE I

| Example No. | X | In the general formula I | | Y | Point °C | Elemental Analysis | | | | | |
|---|---|---|---|---|---|---|---|---|---|---|---|
| | | | | | | Melting | | | Calculated | | |
| | | $R^1$ | $R^2$ | | | C | H | N | C | H | N |
| 8 | S | $—CO_2CH_3$ | $—CH_3$ | S | 115—116 | 33.0 | 2.8 | 12.8 | 33.5 | 2.6 | 12.7 |
| 9 | S | $—CO_2C_2H_5$ | $—CH_3$ | S | 59—60 | 36.2 | 3.5 | 12.1 | 36.0 | 2.9 | 12.3 |
| 10 | S | $—C_6H_5$ | $—C_6H_5$ | S | 114 | No analysis — identified by mass spectrometry | | | | | |
| 11 | O | $—CH_2—CH(CH_3)—CH_2—$ | | S | 54—55 | 45.65 | 4.3 | 15.2 | 45.7 | 4.3 | 15.2 |
| 12 | S | $—CH_2—CH(CH_3)—CH_2—$ | | S | 136.5 | 42.0 | 4.0 | 14.0 | 42.3 | 4.0 | 13.6 |
| 13 | O | $—CH_2—S—CH_2—$ | | S | 97—99 | 31.9 | 2.1 | 14.9 | 31.9 | 1.7 | 14.8 |
| 14 | S | $—CH_2—S—CH_2—$ | | S | 138—140 | 29.4 | 2.0 | 13.7 | 29.5 | 1.7 | 13.6 |
| 15 | O | $—CH(C_6H_5)—CH(C_6H_5)—CH_2—$ | | S | 117 | 67.1 | 4.4 | 8.7 | 67.0 | 4.3 | 8.5 |

TABLE I (continued)

| Example No. | X | In the general formula I $R^1$ — $R^2$ | Y | Point °C | Elemental Analysis Melting C | H | N | Calculated C | H | N |
|---|---|---|---|---|---|---|---|---|---|---|
| 16 | S | $-CH(C_6H_5)-CH(C_6H_5)-CH_2-$ | S | 165—66 | 63.9 | 4.1 | 8.3 | 63.9 | 4.1 | 8.2 |
| 17 | O | 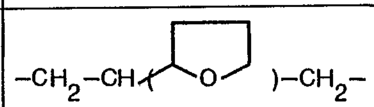 | S | 119 | 50.8 | 3.4 | 11.9 | 50.5 | 3.5 | 11.4 |
| 18 | S | 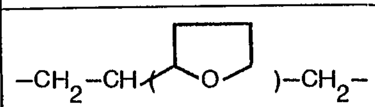 | S | 107 | 47.6 | 3.2 | 11.1 | 47.4 | 3.3 | 11.1 |
| 19 | O | $-CH_2-CH(C_6H_5)-CH(C_6H_5)-$ | S | 193—94 | 63.9 | 4.1 | 8.3 | 63.6 | 4.0 | 8.1 |
| 20 | S | $-CH(C_6H_5)-C(CH_3)_2-CH_2-$ | S | 122 | 57.9 | 4.8 | 9.7 | 57.1 | 4.7 | 9.5 |
| 21 | S | $-CH_2-C(CH_3)_2-CH(C_6H_5)-$ | S | 158 | 57.9 | 4.8 | 9.7 | 56.6 | 4.6 | 9.3 |
| 22 | O | 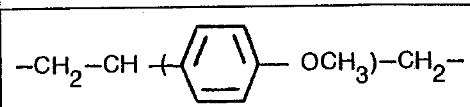 | S | 146 | 56.1 | 4.3 | 10.1 | 56.7 | 4.4 | 10.4 |
| 23 | S | 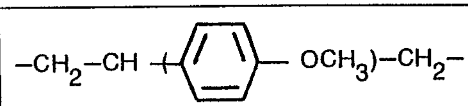 | S | 152—53 | 53.4 | 4.1 | 9.6 | 53.4 | 4.1 | 9.8 |

TABLE I (continued)

| Example No. | X | In the general formula I $R^1$ — $R^2$ | Y | Point °C | Elemental Analysis Melting C | H | N | Calculated C | H | N |
|---|---|---|---|---|---|---|---|---|---|---|
| 24 | O | $-CH_2-S(\overset{O}{\uparrow})-CH_2-$ | S | 183—84 | 29.4 | 2.0 | 13.7 | 29.4 | 1.8 | 13.8 |
| 25 | O | $-CH_2-SO_2-CH_2-$ | S | 167—68 | 27.3 | 1.8 | 12.7 | 27.3 | 1.7 | 12.6 |
| 26 | S | $-CH_2-SO_2-CH_2-$ | S | 185 | 25.4 | 1.7 | 11.9 | 26.0 | 1.7 | 11.5 |
| 27 | O | $-CH[CH(CH_3)_2]-S-CH_2-$ | S | Oil | 41.7 | 4.4 | 12.2 | 41.5 | 4.5 | 12.0 |
| 28 | S | $-CH[CH(CH_3)_2]-S-CH_2-$ | S | Oil | 39.0 | 4.1 | 11.4 | 39.0 | 4.2 | 10.8 |
| 29 | S | $-CH_2-S-CH[CH(CH_3)_2]-$ | S | 76 | 39.0 | 4.1 | 11.4 | 39.1 | 4.0 | 11.2 |
| 30 | S | 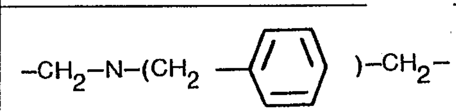 | S | 110—12 | 52.0 | 4.0 | 15.2 | 51.8 | 4.1 | 14.6 |
| 31 | S | 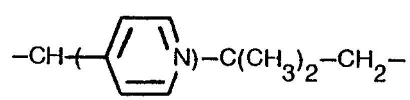 | S | 120—22 | 53.6 | 4.5 | 14.4 | 54.2 | 4.6 | 14.5 |
| 32 | O | $-CH_2-CH_2-CH_2-$ | Se | 104—6 | 33.2 | 2.8 | 12.9 | 33.2 | 2.9 | 13.0 |

TABLE I (continued)

| Example No. | X | In the general formula I R[1] | R[2] | Y | Melting Point °C | Elemental Analysis C | H | N | Calculated C | H | N |
|---|---|---|---|---|---|---|---|---|---|---|---|
| 33 | O | $—CH_2—S—CH_2—$ | | Se | 94—96 | 25.5 | 1.7 | 11.9 | 25.6 | 1.7 | 11.9 |
| 34 | O | $—CH_3$ | $—CH_3$ | Se | 75—76 | 29.3 | 2.9 | 13.7 | 29.4 | 2.9 | 13.7 |
| 35 | O | $—CH_2—CH_2—CH_2—$ | | Te | 165—67 | 27.1 | 2.3 | 10.5 | 27.5 | 2.3 | 10.6 |

Example 36

Herbicidal Activity

To evaluate their herbicidal activity in compositions according to the invention, compounds of the previous Examples were tested using as a representative range of plants: maize, *Zea mays* (MZ); rice, *Oryza sativa* (R); barnyard grass, *Echinochloa crusgalli* (BG); oat, *Avena sativa* (0); linseed, *Linum usitatissimum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing, was drenched with a liquid formulation containing a compound of the invention, and in the foliar spray tests the seedling plants were sprayed with such a formulation.

The soil used in the tests was a prepared horticultural loam.

The formulations used in the tests were prepared by diluting with water, solutions of the test compounds in acetone containing 0.4% by weight of an alkylphenol/ethylene oxide condensate available under the trade name TRITON X—155. The acetone solutions were diluted with water and the resulting formulations applies typically at dosage levels corresponding to 5 kg and/or 1 kg of active material per hectare in a volume equivalent to 650 litres per hectare in the soil spray and foliar spray tests, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedlings plants were used as controls.

The herbicidal effects of the test compounds were assessed visually eleven days aftyer spraying the foliage and drenching the soil and twelve days after spraying the soil, and were recorded on a 0—9 scale. A rating 0 indicates growth as untreated control, a rating 9 indicates death. An increase of 1 unit on the linear scale approximately to a 10% increase in the level of effect.

The results of the tests are set out in Table II below.

TABLE II

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1a | 6 | 7 | 9 | 7 | 9 | 9 | 7 | 5 | 5 | 5 | 5 | 8 | 6 | 9 | 9 | 9 | 8 | 3 | 7 | 9 | 3 | 4 | 5 | 5 | 2 |
| | | | | | | | | | 1 | 3 | 3 | 6 | 3 | 6 | 7 | 7 | 6 | 0 | 4 | 5 | 2 | 2 | 2 | 0 | 0 |
| 1b | 3 | 3 | 4 | 3 | 6 | 7 | 8 | 5 | 5 | 2 | 5 | 6 | 3 | 9 | 9 | 9 | 7 | 2 | 3 | 4 | 0 | 2 | 3 | 6 | 2 |
| | | | | | | | | | 1 | 1 | 3 | 4 | 2 | 8 | 9 | 9 | 6 | 0 | 0 | 0 | 0 | 0 | 0 | 0 | 0 |
| 3a | 5 | 2 | 3 | 3 | 2 | 5 | 5 | 1 | 5 | 3 | 5 | 9 | 6 | 9 | 9 | 9 | 7 | 2 | 5 | 7 | 0 | 3 | 3 | 7 | 2 |
| | | | | | | | | | 1 | 2 | 1 | 8 | 1 | 4 | 6 | 6 | 5 | 0 | 0 | 2 | 0 | 0 | 0 | 4 | 0 |
| 3b | 0 | 4 | 0 | 0 | 9 | 7 | 6 | 0 | 5 | 5 | 7 | 7 | 6 | 9 | 9 | 9 | 9 | 0 | 0 | 5 | 5 | 4 | 2 | 3 | 4 |
| | | | | | | | | | 1 | 3 | 4 | 4 | 2 | 7 | 8 | 9 | 9 | 0 | 0 | 0 | 0 | 0 | 0 | 0 | 4 |
| 5a | 9 | 8 | 9 | 7 | 9 | 9 | 9 | 9 | 5 | 6 | 6 | 9 | 7 | 8 | 9 | 9 | 8 | 4 | 8 | 8 | 3 | 4 | 7 | 9 | 6 |
| | | | | | | | | | 1 | 2 | 0 | 5 | 3 | 6 | 8 | 8 | 4 | 1 | 2 | 2 | 0 | 0 | 2 | 3 | 0 |
| 5b | 0 | 0 | 2 | 0 | 6 | 4 | 8 | 0 | 5 | 4 | 7 | 6 | 5 | 9 | 9 | 9 | 8 | 0 | 0 | 6 | 0 | 6 | 4 | 7 | 2 |
| | | | | | | | | | 1 | 0 | 5 | 5 | 4 | 8 | 9 | 9 | 7 | 0 | 0 | 2 | 0 | 0 | 0 | 0 | 0 |
| 7a | 0 | 0 | 0 | 0 | 0 | 0 | 0 | 0 | 5 | 0 | 0 | 3 | 0 | 3 | 3 | 2 | 2 | 0 | 0 | 0 | 0 | 0 | 0 | 0 | 0 |
| | | | | | | | | | 1 | 0 | 0 | 1 | 0 | 0 | 2 | 1 | 0 | 0 | 0 | 0 | 0 | 0 | 0 | 0 | 0 |

TABLE II (continued)

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 7b | 0 | 0 | 0 | 0 | 0 | 0 | 0 | 0 | 5 | 0 | 2 | 3 | 0 | 4 | 3 | 4 | 3 | 0 | 0 | 0 | 0 | 0 | 0 | 0 | 0 |
| | | | | | | | | | 1 | 0 | 0 | 0 | 0 | 3 | 2 | 3 | 0 | 0 | 0 | 0 | 0 | 0 | 0 | 0 | 0 |
| 8 | — | — | — | — | — | — | — | — | — | | | | | | | | | | | | | | | | |
| | | | | | | | | | 1 | 0 | 0 | 0 | 0 | 0 | 0 | 2 | 0 | 0 | 0 | 0 | 0 | 0 | 0 | 0 | 0 |
| 9 | 0 | 0 | 0 | 0 | 0 | 0 | 0 | 0 | 5 | 2 | 5 | 6 | 4 | 8 | 9 | 8 | 6 | 0 | 0 | 0 | 0 | 0 | 0 | 0 | 0 |
| | | | | | | | | | 1 | 0 | 0 | 2 | 1 | 4 | 4 | 4 | 5 | 0 | 0 | 0 | 0 | 0 | 0 | 0 | 0 |
| 10 | — | — | — | — | — | — | — | — | — | | | | | | | | | | | | | | | | |
| | | | | | | | | | 1 | 0 | 0 | 1 | 0 | 4 | 5 | 0 | 3 | 0 | 0 | 0 | 0 | 0 | 0 | 0 | 0 |
| 11 | 7 | 7 | 6 | 6 | 8 | 9 | 9 | 3 | 5 | 5 | 4 | 9 | 6 | 8 | 9 | 9 | 8 | 5 | 0 | 7 | 3 | 4 | 6 | 3 | 4 |
| | | | | | | | | | 1 | 4 | 3 | 8 | 4 | 7 | 8 | 8 | 5 | 0 | 0 | 3 | 0 | 0 | 3 | 0 | 2 |

TABLE II (continued)

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 12 | 0 | 0 | 2 | 0 | 2 | 3 | 9 | 0 | 5 | 6 | 7 | 9 | 5 | 9 | 9 | 9 | 9 | 0 | 0 | 7 | 0 | 5 | 0 | 6 | 0 |
| | | | | | | | | | 1 | 4 | 5 | 6 | 2 | 7 | 8 | 9 | 7 | 0 | 0 | 0 | 0 | 0 | 0 | 0 | 0 |
| 13 | 8 | 9 | 9 | 8 | 9 | 9 | 9 | 9 | 5 | 8 | 9 | 9 | 8 | 9 | 9 | 9 | 9 | 8 | — | 9 | 7 | 7 | 9 | 9 | 8 |
| | | | | | | | | | 1 | 5 | 8 | 9 | 6 | 8 | 9 | 9 | 8 | 5 | — | 8 | 5 | 5 | 8 | 8 | 4 |
| 14 | 0 | 0 | 0 | 0 | 0 | 0 | 0 | 0 | 5 | 3 | 6 | 8 | 4 | 9 | 9 | 9 | 6 | 3 | — | 3 | 0 | 0 | 3 | 4 | 0 |
| | | | | | | | | | 1 | 1 | 5 | 7 | 4 | 9 | 9 | 9 | 5 | 0 | — | 0 | 0 | 0 | 1 | 2 | 0 |
| 15 | 0 | 0 | 0 | 0 | 0 | 0 | 0 | 0 | 5 | 0 | 0 | 3 | 1 | 4 | 6 | 4 | 3 | 0 | 0 | 0 | 0 | 0 | 0 | 0 | 0 |
| | | | | | | | | | 1 | 0 | 0 | 0 | 0 | 1 | 4 | 0 | 0 | 0 | 0 | 0 | 0 | 0 | 0 | 0 | 0 |
| 16 | 0 | 0 | 0 | 0 | 0 | 0 | 0 | 0 | 5 | 0 | 0 | 6 | 2 | 7 | 7 | 6 | 6 | 0 | 0 | 0 | 0 | 0 | 0 | 0 | 0 |
| | | | | | | | | | 1 | 0 | 0 | 0 | 0 | 4 | 3 | 2 | 2 | 0 | 0 | 0 | 0 | 0 | 0 | 0 | 0 |
| 17 | 0 | 0 | 0 | 0 | 0 | 0 | 0 | 0 | — | | | | | | | | | | | | | | | | |
| | | | | | | | | | 1 | 0 | 0 | 7 | 2 | 3 | 2 | 0 | 0 | 0 | 0 | 0 | 0 | 0 | 0 | 0 | 0 |
| 18 | 0 | 0 | 0 | 0 | 0 | 0 | 0 | 0 | 5 | 4 | 3 | 7 | 4 | 8 | 9 | 9 | 6 | 0 | 0 | 0 | 0 | 0 | 0 | 0 | 0 |
| | | | | | | | | | 1 | 2 | 1 | 5 | 2 | 8 | 8 | 7 | 6 | 0 | 0 | 0 | 0 | 0 | 0 | 0 | 0 |

TABLE II (continued)

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 19 | 0 | 0 | 0 | 0 | 0 | 0 | 0 | 0 | 5 | 0 | 0 | 0 | 0 | 3 | 2 | 4 | 2 | 0 | 0 | 0 | 0 | 0 | 0 | 0 | 0 |
| | | | | | | | | | 1 | 0 | 0 | 0 | 0 | 1 | 0 | 2 | 1 | 0 | 0 | 0 | 0 | 0 | 0 | 0 | 0 |
| 20 | 0 | 0 | 0 | 0 | 0 | 0 | 0 | 0 | 5 | 3 | 0 | 6 | 3 | 8 | 9 | 4 | 2 | 0 | 0 | 0 | 0 | 0 | 0 | 0 | 0 |
| | | | | | | | | | 1 | 1 | 0 | 4 | 1 | 7 | 8 | 2 | 1 | 0 | 0 | 0 | 0 | 0 | 0 | 0 | 0 |
| 21 | 0 | 0 | 0 | 0 | 0 | 0 | 0 | 0 | 5 | 4 | 0 | 6 | 4 | 7 | 9 | 4 | 8 | 0 | 0 | 0 | 0 | 0 | 0 | 0 | 0 |
| | | | | | | | | | 1 | 2 | 0 | 6 | 1 | 6 | 8 | 2 | 6 | 0 | 0 | 0 | 0 | 0 | 0 | 0 | 0 |
| 22 | 0 | 0 | 0 | 0 | 0 | 0 | 0 | 0 | 5 | 0 | 0 | 0 | 0 | 0 | 5 | 3 | 2 | 0 | 0 | 0 | 0 | 0 | 0 | 0 | 0 |
| | | | | | | | | | 1 | 0 | 0 | 0 | 0 | 0 | 3 | 0 | 0 | 0 | 0 | 0 | 0 | 0 | 0 | 0 | 0 |
| 23 | 0 | 0 | 0 | 0 | 0 | 0 | 0 | 0 | — | | | | | | | | | | | | | | | | |
| | | | | | | | | | 1 | 0 | 0 | 0 | 0 | 6 | 6 | 4 | 0 | 0 | 0 | 0 | 0 | 0 | 0 | 0 | 0 |

TABLE II (continued)

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 24 | 7 | 8 | 9 | 7 | 9 | 9 | 9 | 9 | 5 | 8 | 9 | 9 | 8 | 9 | 9 | 9 | 9 | 8 | — | 9 | 7 | 6 | 8 | 8 | 0 |
| | | | | | | | | | 1 | 6 | 7 | 9 | 5 | 7 | 9 | 9 | 8 | 6 | — | 9 | 5 | 2 | 5 | 4 | 0 |
| 25 | 8 | 5 | 6 | 8 | 7 | 9 | 0 | 9 | 5 | 7 | 8 | 9 | 8 | 9 | 9 | 9 | 9 | 3 | — | 7 | 3 | 4 | 7 | 0 | 0 |
| | | | | | | | | | 1 | 4 | 6 | 8 | 6 | 8 | 9 | 5 | 7 | 3 | — | 5 | 2 | 0 | 3 | 0 | 0 |
| 26 | 0 | 0 | 0 | 0 | 0 | 0 | 0 | 0 | 5 | 3 | 0 | 2 | 2 | 5 | 5 | 3 | 3 | 0 | 0 | 0 | 0 | 0 | 0 | 0 | 0 |
| | | | | | | | | | 1 | 0 | 0 | 1 | 0 | 2 | 2 | 2 | 2 | 0 | 0 | 0 | 0 | 0 | 0 | 0 | 0 |
| 27 | 8 | 6 | 9 | 8 | 9 | 9 | 9 | 8 | 5 | 7 | 6 | 9 | 8 | 9 | 9 | 9 | 9 | 5 | 7 | 9 | 7 | 9 | 8 | 8 | 4 |
| | | | | | | | | | 1 | 2 | 2 | 8 | 5 | 7 | 9 | 8 | 8 | 3 | 3 | 8 | 3 | 6 | 6 | 6 | 3 |
| 28 | 0 | 0 | 2 | 0 | 4 | 0 | 3 | 2 | 5 | 4 | 9 | 6 | 6 | 8 | 9 | 9 | 9 | 0 | 0 | 0 | 0 | 0 | 0 | 0 | 0 |
| | | | | | | | | | 1 | 2 | 4 | 4 | 3 | 7 | 9 | 9 | 8 | 0 | 0 | 0 | 0 | 0 | 0 | 0 | 0 |

TABLE II (continued)

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 29 | 0 | 0 | 0 | 0 | 0 | 0 | 0 | 0 | 5 | 5 | 5 | 6 | 5 | 8 | 9 | 9 | 9 | 0 | 0 | 0 | 0 | 0 | 0 | 0 | 0 |
| | | | | | | | | | 1 | 3 | 3 | 4 | 2 | 7 | 9 | 8 | 5 | 0 | 0 | 0 | 0 | 0 | 0 | 0 | 0 |
| 30 | 0 | 0 | 0 | 0 | 0 | 0 | 0 | 0 | 5 | — | — | — | — | — | — | — | — | — | — | — | — | — | — | — | — |
| | | | | | | | | | 1 | 3 | 0 | 3 | 2 | 5 | 4 | 3 | 3 | 0 | 0 | 0 | 0 | 0 | 0 | 0 | 0 |
| 31 | 0 | 0 | 0 | 0 | 0 | 0 | 0 | 0 | 5 | 3 | 4 | 7 | 4 | 9 | 9 | 9 | 8 | 0 | 0 | 0 | 0 | 0 | 0 | 0 | 0 |
| | | | | | | | | | 1 | 1 | 2 | 3 | 2 | 4 | 7 | 5 | 4 | 0 | 0 | 0 | 0 | 0 | 0 | 0 | 0 |
| 32 | 3 | 0 | 4 | 0 | 4 | 7 | 6 | 7 | 5 | 5 | 5 | 9 | 8 | 8 | 9 | 9 | 9 | 3 | 0 | 6 | 4 | 3 | 3 | 9 | 2 |
| | | | | | | | | | 1 | 4 | 2 | 5 | 4 | 6 | 9 | 9 | 7 | 0 | 0 | 3 | 0 | 0 | 0 | 2 | 0 |
| 33 | 8 | 6 | 6 | 5 | 5 | 6 | 6 | 8 | 5 | 6 | 5 | 8 | 7 | 8 | 9 | 9 | 9 | 5 | 5 | 7 | 4 | 5 | 6 | 7 | 2 |
| | | | | | | | | | 1 | 2 | 3 | 7 | 6 | 7 | 8 | 8 | 6 | 0 | 0 | 6 | 0 | 2 | 3 | 2 | 0 |
| 34 | 9 | 9 | 9 | 8 | 9 | 9 | 9 | 9 | 5 | 7 | 6 | 9 | 6 | 9 | 9 | 9 | 9 | 7 | 9 | 9 | 5 | 3 | 7 | 9 | 4 |
| | | | | | | | | | 1 | 5 | 4 | 9 | 5 | 9 | 8 | 9 | 9 | 2 | 6 | 5 | 0 | 0 | 3 | 7 | 0 |
| 35 | 2 | 0 | 3 | 3 | 8 | 6 | 3 | 7 | 5 | 4 | 6 | 8 | 6 | 9 | 9 | 9 | 9 | 0 | 0 | 0 | 0 | 0 | 4 | 0 | 0 |
| | | | | | | | | | 1 | 3 | 4 | 7 | 5 | 8 | 9 | 9 | 9 | 0 | 0 | 0 | 0 | 0 | 0 | 0 | 0 |

**Claims for the Contracting States: BE CH DE FR GB IT LI NL SE**

1. A herbicidal composition which comprises at least two carriers, at least one of which is a surface-active agent, together with, as active ingredient, a compound of the general formula I or where possible an acid addition salt thereof:

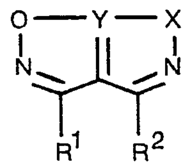

(I)

in which Y represents a sulphur, selenium or tellurium atom; $R^1$ and $R^2$ each independently represent an alkyl group of up to 6 carbon atoms, a phenyl group or a group

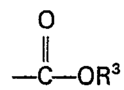

wherein $R^3$ is an alkyl group of up to 6 carbon atoms; or $R^1$ and $R^2$ together represent an alkylene linkage containing up to 4 carbon atoms in the chain or an alkylene linkage containing up to 3 carbon atoms and one heteroatom selected from sulphur, nitrogen and oxygen in the chain, each linkage being optionally substituted with up to 3 alkyl groups each containing up to 12 carbon atoms, up to 3 phenyl groups, a benzyl group, an acyl group of up to 12 carbon atoms, a pyridyl group or salt thereof, an alkoxy substituted phenyl group containing up to 6 carbon atoms in the alkyl portion of the alkoxy moiety or a furyl group, and when the heteroatom is sulphur the oxidised derivatives thereof; and X represents an oxygen atom or, when Y represents a sulphur atom, X may alternatively represent a sulphur atom.

2. A composition as claimed in claim 1 in which the active ingredient is a compound of the formula II or where possible an acid addition salt thereof:

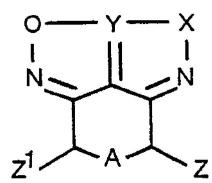

(II)

wherein X and Y have the meanings given in claim 1, Z represents a hydrogen atom, an alkyl group of up to 12 carbon atoms, a phenyl group, or pyridyl group or salt thereof or a benzyl group; $Z^1$ represents a hydrogen atom, an alkyl group of up to 6 carbon atoms, a pyridyl group or salt thereof or a phenyl group and A represents a group:

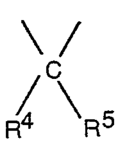 , 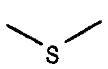 , 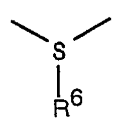 , 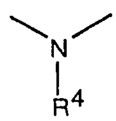 , 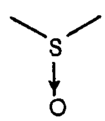 , 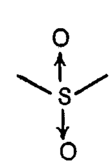 or 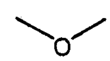

wherein $R^4$ represents a hydrogen atom, an alkyl group of up to 12 carbon atoms, a benzyl group or a pyridyl group or salt thereof, or a group

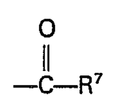

wherein $R^7$ is an alkyl or alkenyl group of up to 12 carbon atoms, an aryl group of up to 12 carbon atoms, an aralkyl group of up to 9 carbon atoms or an alkaryl group of up to 9 carbon atoms, $R^5$ represents a hydrogen atom or an alkyl group of up to 6 carbon atoms and $R^6$ represents an alkyl group of up to 12 carbon atoms or a benzyl group, with the proviso that both of Z and $Z^1$ cannot simultaneously be a pyridyl group or salt thereof.

3. A composition as claimed in claim 2, in which Z and $Z^1$ each represent a hydrogen atom or an alkyl group of up to 3 carbon atoms and A is a group:

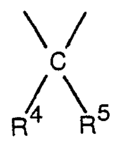 , 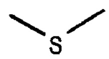 or 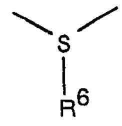

wherein $R^4$ is a hydrogen atom or an alkyl group of up to 9 carbon atoms, $R^5$ is a hydrogen atom or an alkyl group of up to 3 carbon atoms and $R^6$ is an alkyl group of up to 9 carbon atoms.

4. A composition as claimed in claim 3 in which $R^4$ and $R^5$ each represent a hydrogen atom or a methyl group and $R^6$ is a methyl group.

5. A composition as claimed in any one of the preceding claims wherein Y represents a sulphur atom.

6. A compound of the general formula I or where possible an acid addition salt thereof as defined in claim 1, wherein Y is sulphur with the proviso that one of $R^1$ and $R^2$ cannot be methyl when the other is ethoxycarbonyl and further provided that when $R^1$ and $R^2$ together represent an alkylene linkage of 3 or 4 carbon atoms the carbon chain of the linkage must be substituted with at least one of the groups indicated as optional substituents and when the alkylene linkage contains 3 carbon atoms the middle carbon atom must not be substituted with two methyl groups if the other two carbon atoms are unsubstituted.

7. A compound of formula II or where possible an acid addition salt thereof as defined in claims 2 to 4 with the proviso that both of Z and $X^1$ cannot simultaneously be hydrogen when Y represents a group

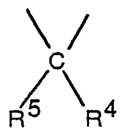

and $R^4$ and $R^5$ are the same, each representing hydrogen or methyl.

8. A process for the preparation of a compound as claimed in claim 6, which comprises reacting a dioxime of the general formula:

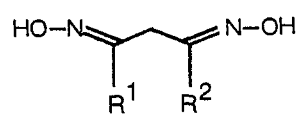

(III)

with sulphur mono chloride and/or sulphur dichloride to afford a reaction product containing at least one compound according to formula I wherein X is oxygen or sulphur and optionally a second compound according to formula I which differs from the first compound according to formula I only in the nature of the X substituent, X representing oxygen in one compound and X representing sulphur in the other compound and, if the two compounds according to formula I are present in the reaction product, optionally separating the two compounds from the reaction product as individual components.

9. A method of combating undesired plant growth at a locus, which comprises treating the locus with a compound as claimed in claim 6 or 7 or a composition as claimed in any of claims 1 to 5.

**Claims for the Contracting State: AT**

1. A herbicidal composition which comprises at least two carriers, at least one of which is a surface-active agent, together with, as active ingredient, a compound of the general formula I or where possible an acid addition salt thereof:

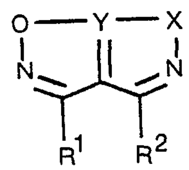

(I)

in which Y represents a sulphur, selenium or tellurium atom; $R^1$ and $R^2$ each independently represent an alkyl group of up to 6 carbon atoms, a phenyl group or a group

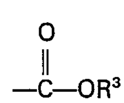

wherein $R^3$ is an alkyl group of up to 6 carbon atoms; or $R^1$ and $R^2$ together represent an alkylene linkage

containing up to 4 carbon atoms in the chain or an alkylene linkage containing up to 3 carbon atoms and one heteroatom selected from sulphur, nitrogen and oxygen in the chain, each linkage being optionally substituted with up to 3 alkyl groups each containing up to 12 carbon atoms, up to 3 phenyl groups, a benzyl group, an acyl group of up to 12 carbon atoms, a pyridyl group or salt thereof, an alkoxy substituted phenyl group containing up to 6 carbon atoms in the alkyl portion of the alkoxy moiety or a furyl group, and when the heteroatom is sulphur the oxidised derivatives thereof; and X represents an oxygen atom or, when Y represents a sulphur atom, X may alternatively represent a sulphur atom.

2. A composition as claimed in claim 1 in which the active ingredient is a compound of the formula II or where possible an acid addition salt thereof:

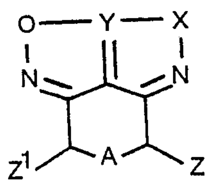

(II)

wherein X and Y have the meanings given in claim 1, Z represents a hydrogen atom, an alkyl group of up to 12 carbon atoms, a phenyl group, or pyridyl group or salt thereof or a benzyl group; $Z^1$ represents a hydrogen atom, an alkyl group of up to 6 carbon atoms, a pyridyl group or salt thereof or a phenyl group and A represents a group:

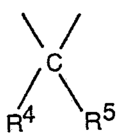 , 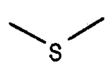 , 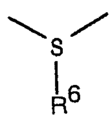 , 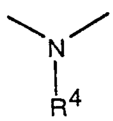 , 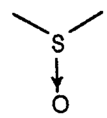 , 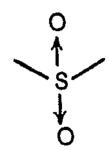 or 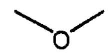

wherein $R^4$ represents a hydrogen atom, an alkyl group of up to 12 carbon atoms, a benzyl group or a pyridyl group or salt thereof, or a group

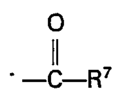

wherein $R^7$ is an alkyl or alkenyl group of up to 12 carbon atoms, an aryl group of up to 12 carbon atoms, an aralkyl group of up to 9 carbon atoms or an alkaryl group of up to 9 carbon atoms, $R^5$ represents a hydrogen atom or an alkyl group of up to 6 carbon atoms and $R^6$ represents an alkyl group of up to 12 carbon atoms or a benzyl group, with the proviso that both of Z and $Z^1$ cannot simultaneously be a pyridyl group or salt thereof.

3. A composition as claimed in claim 2, in which Z and $Z^1$ each represent a hydrogen atom or an alkyl group of up to 3 carbon atoms and A is a group:

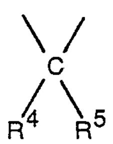 , 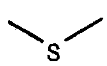 or 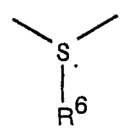

wherein $R^4$ is a hydrogen atom or an alkyl group of up to 9 carbon atoms, $R^5$ is a hydrogen atom or an alkyl group of up to 3 carbon atoms and $R^6$ is an alkyl group of up to 9 carbon atoms.

4. A composition as claimed in claim 3 in which $R^4$ and $R^5$ each represent a hydrogen atom or a methyl group and $R^6$ is a methyl group.

5. A composition as claimed in any one of the preceding claims wherein Y represents a sulphur atom.

6. A process for the preparation of a compound of formula I as defined by any of claims 1 to 5, which comprises reacting a dioxime of the general formula:

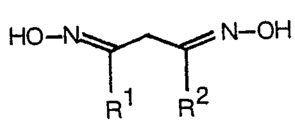

(III)

with sulphur mono chloride and/or sulphur dichloride to afford a reaction product containing at least one

compound according to formula I wherein X is oxygen or sulphur and optionally a second compound according to formula I which differs from the first compound according to formula I only in the nature of the X substituent, X representing oxygen in one compound and X representing sulphur in the other compound and, if the two compounds according to formula I are present in the reaction product, optionally separating the two compounds from the reaction product as individual components.

7. A method of combating undesired plant growth at a locus, which comprises treating the locus with a compound or composition as defined by any of claims 1 to 5.

**Patentansprüche für die Vertragsstaaten: BE CH DE FR GB IT LI NL SE**

1. Herbizide Zusammensetzung, umfassend wenigstens zwei Träger, von denen wenigstens einer ein oberflächenaktives Mittel ist, zusammen mit, als aktiver Bestandteil, einer Verbindung der allgemeinen Formel I oder, falls dies möglich ist, einem Säureadditionssalz hievon:

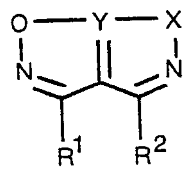

(I)

worin Y ein Schwefel-, Selen- oder Telluratom bedeutet; $R^1$ und $R^2$ unabhängig voneinander jeweils eine Alkylgruppe mit bis zu 6 Kohlenstoffatomen, eine Phenylgruppe oder eine Gruppe

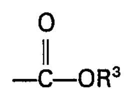

darstellen, worin $R^3$ eine Alkylgruppe mit bis zu 6 Kohlenstoffatomen bedeutet; oder $R^1$ und $R^2$ gemeinsam eine Alkylenverknüpfung mit bis zu 4 Kohlenstoffatomen in der Kette oder eine Alkylenverknüpfung mit bis zu 3 Kohlenstoffatomen und einem unter Schwefel, Stickstoff und Sauerstoff ausgewählten Heteroatom in der Kette bedeuten, wobei jede Verknüpfung gegebenenfalls durch bis zu 3 Alkylgruppen mit jeweils bis zu 12 Kohlenstoffatomen, durch bis zu 3 Phenylgruppen, eine Benzylgruppe, eine Acylgruppe mit bis zu 12 Kohlenstoffatomen, eine Pyridylgruppe oder ein Salz hievon, eine Alkoxysubstituierte Phenylgruppe mit bis zu 6 Kohlenstoffatomen im Alkylteil des Alkoxyrestes oder eine Furylgruppe und, wenn das Heteroatom Schwefel ist, durch die Oxidationsderivate hievon substituiert ist; und X ein Sauerstoffatom darstellt oder, falls Y ein Schwefelatom bedeutet, X wahlweise ein Schwefelatom bedeuten kann.

2. Zusammensetzung nach Anspruch 1, worin der aktive Bestandteil eine Verbindung der Formel II oder, falls möglich, ein Säureadditionssalz hievon ist:

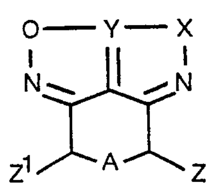

(II)

worin X und Y die in der allgemeinen Formel I angegebenen Bedeutungen besitzen, Z ein Wasserstoffatom, eine Alkylgruppe mit bis zu 12 Kohlenstoffatomen, eine Phenylgruppe, eine Pyridylgruppe oder ein Salz hievon oder eine Benzylgruppe darstellt; $Z^1$ ein Wasserstoffatom, eine Alkylgruppe mit bis zu 6 Kohlenstoffatomen, eine Pyridylgruppe oder ein Salz hievon oder eine Phenylgruppe bedeutet und A eine Gruppe

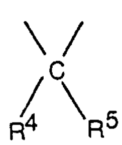 , 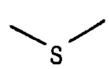 , 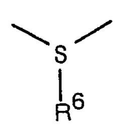 , 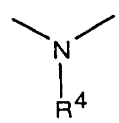 , 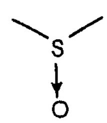 , 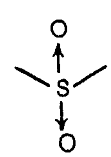 oder 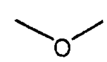

darstellt, worin $R^4$ ein Wasserstoffatom, eine Alkylgruppe mit bis zu 12 Kohlenstoffatomen, eine Benzylgruppe oder eine Pyridylgruppe oder ein Salz hievon oder eine Gruppe

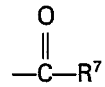

bedeutet, worin $R^7$ eine Alkyl- oder Alkenylgruppe mit bis zu 12 Kohlenstoffatomen, eine Arylgruppe mit bis zu 12 Kohlenstoffatomen, eine Aralkylgruppe mit bis zu 9 Kohlenstoffatomen oder eine Alkarylgruppe mit bis zu 9 Kohlenstoffatomen darstellt, $R^5$ ein Wasserstoffatom oder eine Alkylgruppe mit bis zu 6 Kohlenstoffatomen bedeutet und $R^6$ eine Alkylgruppe mit bis zu 12 Kohlenstoffatomen oder eine Benzylgruppe darstellt, mit der Maßgabe, daß beide Gruppen Z und $Z^1$ nicht gleichzeitig eine Pyridylgruppe oder ein Salz hievon darstellen können.

3. Zusammensetzung nach Anspruch 2, worin Z und $Z^1$ jeweils ein Wasserstoffatom oder eine Alkylgruppe mit bis zu 3 Kohlenstoffatomen bedeuten und A für eine Gruppe

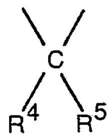 , 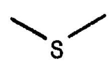 oder 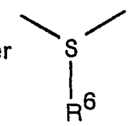

steht, worin $R^4$ ein Wasserstoffatom oder eine Alkylgruppe mit bis zu 9 Kohlenstoffatomen bedeutet, $R^5$ ein Wasserstoffatom oder eine Alkylgruppe mit bis zu 3 Kohlenstoffatomen darstellt und $R^6$ eine Alkylgruppe mit bis zu 9 Kohlenstoffatomen bedeutet.

4. Zusammensetzung nach Anspruch 3, worin $R^4$ und $R^5$ jeweils ein Wasserstoffatom oder eine Methylgruppe bedeuten und $R^6$ eine Methylgruppe darstellt.

5. Zusammensetzung nach einem der vorstehenden Ansprüche, worin Y ein Schwefelatom darstellt.

6. Verbindung der allgemeinen Formel I oder, falls dies möglich ist, ein Säureadditionssalz, gemäß Definition in Anspruch 1, worin Y für Schwefel steht, mit der Maßgabe, daß einer der Reste $R^1$ und $R^2$ nicht Methyl sein kann, wenn der andere für Ethoxycarbonyl steht, und mit der weiteren Maßgabe, daß dann, wenn $R^1$ und $R^2$ gemeinsam eine Alkylenverknüpfung von 3 oder 4 Kohlenstoffatomen bedeuten, die Kohlenstoffkette der Verknüpfung durch wenigstens eine der Gruppen substituiert sein muß, die als fakultative Substituenten angegeben sind, und wenn die Alkylenverknüpfung 3 Kohlenstoffatome enthält, das mittlere Kohlenstoffatom nicht durch 2 Methylgruppen substituiert sein darf, wenn die beiden anderen Kohlenstoffatome unsubstituiert sind.

7. Verbindung nach Formel II oder, falls dies möglich ist, ein Säureadditionssalz hievon, gemäß Definition in den Ansprüchen 2 bis 4, mit der Maßgabe, daß beide Reste Z und $Z^1$ nicht gleichzeitig Wasserstoff sein können, wenn A für eine Gruppe

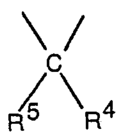

steht und $R^4$ und $R^5$ die gleiche Bedeutung haben und jeweils Wasserstoff oder Methyl darstellen.

8. Verfahren zur Herstellung einer Verbindung, wie in Anspruch 6 beansprucht, welches ein Umsetzen eines Dioxims der allgemeinen Formel:

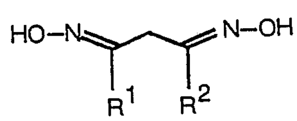

(III)

mit Schwefelmonochlorid und/oder Schwefeldichlorid zur Ausbildung eines Reaktionsproduktes, das wenigstens eine Verbindung gemäß Formel I, worin X Sauerstoff oder Schwefel bedeutet, und gegebenenfalls eine zweite Verbindung gemäß Formel I enthält, die sich von der ersten Verbindung gemäß Formel I nur in der Art des Substituenten X unterscheidet, wobei X in einer Verbindung Sauerstoff bedeutet und X in der anderen Verbindung Schwefel darstellt, und, falls die beiden Verbindungen gemäß der Formel I im Reaktionsprodukt vorliegen, gegebenenfalls ein Abtrennen der beiden Verbindungen aus dem Reaktionsprodukt als individuelle Komponenten umfasst.

9. Verfahren zur Bekämpfung von unerwünschtem Pflanzenwuchs an einem Ort, welches ein Behandeln des Ortes mit einer Verbindung wie in Anspruch 6 oder 7 beansprucht, oder mit einer Zusammensetzung, wie in einem der Ansprüche 1—5 beansprucht, umfasst.

**Patentansprüche für den Vertragsstaat: AT**

1. Herbizide Zusammensetzung, umfassend wenigstens zwei Träger, von denen wenigstens einer ein oberflächenaktives Mittel ist, zusammen mit, als aktiver Bestandteil, einer Verbindung der allgemeinen Formel I oder, falls dies möglich ist, einem Säureadditionssalz hievon:

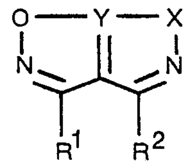

(I)

worin Y ein Schwefel-, Selen- oder Telluratom bedeutet; $R^1$ und $R^2$ unabhängig voneinander jeweils eine Alkylgruppe mit bis zu 6 Kohlenstoffatomen, eine Phenylgruppe oder eine Gruppe

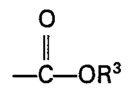

darstellen, worin $R^3$ eine Alkylgruppe mit bis zu 6 Kohlenstoffatomen bedeutet; oder $R^1$ und $R^2$ gemeinsam eine Alkylenverknüpfung mit bis zu 4 Kohlenstoffatomen in der Kette oder eine Alkylenverknüpfung mit bis zu 3 Kohlenstoffatomen und einem unter Schwefel, Stickstoff und Sauerstoff ausgewählten Heteroatom in der Kette bedeuten, wobei jede Verknüpfung gegebenenfalls durch bis zu 3 Alkylgruppen mit jeweils bis zu 12 Kohlenstoffatomen, durch bis zu 3 Phenylgruppen, eine Benzylgruppe, eine Acylgruppe mit bis zu 12 Kohlenstoffatomen, eine Pyridylgruppe oder ein Salz hievon, eine Alkoxysubstituierte Phenylgruppe mit bis zu 6 Kohlenstoffatomen im Alkylteil des Alkoxyrestes oder eine Furylgruppe und, wenn das Heteroatom Schwefel ist, durch die Oxidationsderivate hievon substituiert ist; und X ein Sauerstoffatom darstellt oder, falls Y ein Schwefelatom bedeutet, X wahlweise ein Schwefelatom bedeuten kann.

2. Zusammensetzung nach Anspruch 1, worin der aktive Bestandteil eine Verbindung der Formel II oder, falls möglich, ein Säureadditionssalz hievon ist:

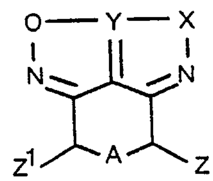

(II)

worin X und Y die in der allgemeinen Formel I angegebenen Bedeutungen besitzen, Z ein Wasserstoffatom, eine Alkylgruppe mit bis zu 12 Kohlenstoffatomen, eine Phenylgruppe, eine Pyridylgruppe oder ein Salz hievon oder eine Benzylgruppe darstellt; $Z^1$ ein Wasserstoffatom, eine Alkylgruppe mit bis zu 6 Kohlenstoffatomen, eine Pyridylgruppe oder ein Salz hievon oder eine Phenylgruppe bedeutet und A eine Gruppe

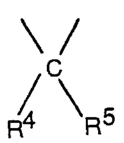 , 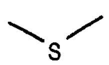 , 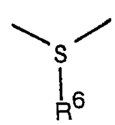 , 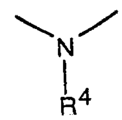 , 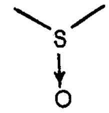 , 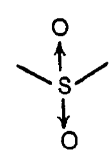 oder 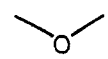

darstellt, worin $R^4$ ein Wasserstoffatom, eine Alkylgruppe mit bis zu 12 Kohlenstoffatomen, eine Benzylgruppe oder eine Pyridylgruppe oder ein Salz hievon oder eine Gruppe

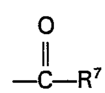

bedeutet, worin $R^7$ eine Alkyl- oder Alkenylgruppe mit bis zu 12 Kohlenstoffatomen, eine Arylgruppe mit bis zu 12 Kohlenstoffatomen, eine Aralkylgruppe mit bis zu 9 Kohlenstoffatomen oder eine Alkarylgruppe mit bis zu 9 Kohlenstoffatomen darstellt, $R^5$ ein Wasserstoffatom oder eine Alkylgruppe mit bis zu 6 Kohlenstoffatomen bedeutet und $R^6$ eine Alkylgruppe mit bis zu 12 Kohlenstoffatomen oder eine Benzylgruppe darstellt, mit der Maßgabe, daß beide Gruppen Z und $Z^1$ nicht gleichzeitig eine Pyridylgruppe oder ein Salz hievon darstellen können.

3. Zusammensetzung nach Anspruch 2, worin Z und $Z^1$ jeweils ein Wasserstoffatom oder eine Alkylgruppe mit bis zu 3 Kohlenstoffatomen bedeuten und A für eine Gruppe

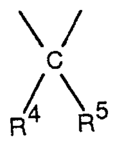 , 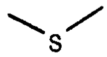 oder 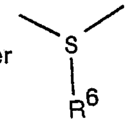

steht, worin $R^4$ ein Wasserstoffatom oder eine Alkylgruppe mit bis zu 9 Kohlenstoffatomen bedeutet, $R^5$ ein Wasserstoffatom oder eine Alkylgruppe mit bis zu 3 Kohlenstoffatomen darstellt und $R^6$ eine Alkylgruppe mit bis zu 9 Kohlenstoffatomen bedeutet.

4. Zusammensetzung nach Anspruch 3, worin $R^4$ und $R^5$ jeweils ein Wasserstoffatom oder eine Methylgruppe bedeuten und $R^6$ eine Methylgruppe darstellt.

5. Zusammensetzung nach einem der vorstehenden Ansprüche, worin Y ein Schwefelatom darstellt.

6. Verfahren zur Herstellung einer Verbindung der Formel I, wie in einem der Ansprüche 1 bis 5 definiert, welches ein Umsetzen eines Dioxims der allgemeinen Formel:

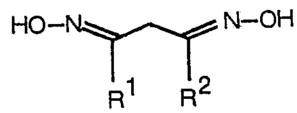

(III)

mit Schwefelmonochlorid und/oder Schwefeldichlorid zur Ausbildung eines Reaktionsproduktes, das wenigstens eine Verbindung gemäß Formel I, worin X Sauerstoff oder Schwefel bedeutet, und gegebenenfalls eine zweite Verbindung gemäß Formel I enthält, die sich von der ersten Verbindung gemäß Formel I nur in der Art des Substituenten X unterscheidet, wobei X in einer Verbindung Sauerstoff bedeutet und X in der anderen Verbindung Schwefel darstellt, und, falls die beiden Verbindungen gemäß der Formel I im Reaktionsprodukt vorliegen, gegebenenfalls ein Abtrennen der beiden Verbindungen aus dem Reaktionsprodukt als individuelle Komponenten umfasst.

7. Verfahren zur Bekämpfung von unerwünschtem Pflanzenwuchs an einem Ort, welches ein Behandeln des Ortes mit einer Verbindung oder einer Zusammensetzung, wie in einem der Ansprüche 1 bis 5 definiert, umfasst.

**Revendications pour les Etats contractants: BE CH DE FR GB IT LI NL SE**

1. Une composition herbicide qui comprend au moins deux véhicules, dont l'un au moins est un agent tensio-actif, en même temps que, comme ingrédient actif, un composé de la formule générale I ou, quand c'est possible, un sel d'addition d'acide d'un tel composé:

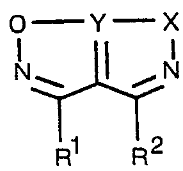

(I)

où Y représente un atome de soufre, de sélénium ou de tellure; $R^1$ et $R^2$ représentent chacun indépendamment un groupe alcoyle ayant jusqu'à 6 atomes de carbone, un groupe phényle ou un groupe

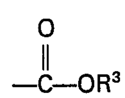

où $R^3$ est un groupe alcoyle ayant jusqu'à 6 atomes de carbone; ou $R^1$ et $R^2$ représentent ensemble une liaison alcoylène contenant jusqu'à 4 atomes de carbone dans la chaîne ou une liaison alcoylène contenant jusqu'à 3 atomes de carbone et un hétéro-atome choisi parmi le soufre, l'azote et l'oxygène dans la chaîne, chaque liaison étant éventuellement substituée par jusqu'à 3 groupes alcoyle contenant chacun jusqu'à 12 atomes de carbone, jusqu'à 3 groupes phényle, un groupe benzyle, un groupe acyle ayant jusqu'à 12 atomes de carbone, un groupe pyridyle ou un sel correspondant, un groupe phényle alcoxylé contenant jusqu'à 6 atomes de carbone dans la portion alcoyle de la portion alcoxy ou un groupe furyle et, quand l'hétéro-atome est du soufre, ses dérivés oxydés; et X représente un atome d'oxygène ou, quand Y

représente un atome de soufre, X peut en variante représenter un atome de soufre.

2. Une composition selon la revendication 1 dans laquelle l'ingrédient actif est un composé de la formule II ou, quand c'est possible, un sel d'addition d'acide d'un tel composé:

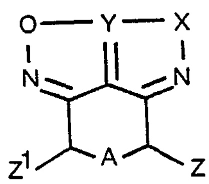

(II)

où X et Y ont les significations indiquées dans la revendication 1, Z représente un atome d'hydrogène, un groupe alcoyle ayant jusqu'à 12 atomes de carbone, un groupe phényle ou un groupe pyridyle ou un sel correspondant ou un groupe benzyle; $Z^1$ représente un atome d'hydrogène, un groupe alcoyle ayant jusqu'à 6 atomes de carbone, un groupe pyridyle ou un sel correspondant ou un groupe phényle et A représente un groupe:

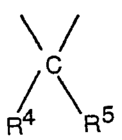 , 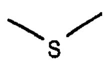 , 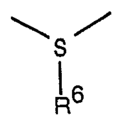 , 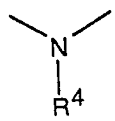 , 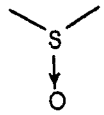 , 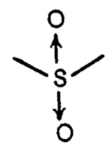 ou 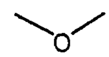

où $R^4$ représente un atome d'hydrogène, un groupe alcoyle ayant jusqu'à 12 atomes de carbone, un groupe benzyle ou un groupe pyridyle ou un sel correspondant, ou un groupe

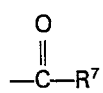

où $R^7$ est un groupe alcoyle ou alcényle ayant jusqu'à 12 atomes de carbone, un groupe aryle ayant jusqu'à 12 atomes de carbone, un groupe aralcoyle ayant jusqu'à 9 atomes de carbone ou un groupe alcaryle ayant jusqu'à 9 atomes de carbone, $R^5$ représente un atome d'hydrogène ou un groupe alcoyle ayant jusqu'à 6 atomes de carbone et $R^6$ représente un groupe alcoyle ayant jusqu'à 12 atomes de carbone ou un groupe benzyle, avec la condition que Z et $Z^1$ ne peuvent pas être tous deux simultanément un groupe pyridyle ou un sel correspondant.

3. Une composition selon la revendication 2, dans laquelle Z et $Z^1$ représentent chacun un atome d'hydrogène ou un groupe alcoyle ayant jusqu'à 3 atomes de carbone et A est un groupe:

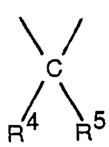 , 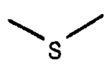 ou 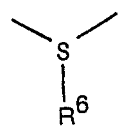

où $R^4$ est un atome d'hydrogène ou un groupe alcoyle ayant jusqu'à 9 atomes de carbone, $R^5$ est un atome d'hydrogène ou un groupe alcoyle ayant jusqu'à 3 atomes de carbone et $R^6$ est un groupe alcoyle ayant jusqu'à 9 atomes de carbone.

4. Une composition selon la revendication 3, dans laquelle $R^4$ et $R^5$ représentent chacun un atome d'hydrogène ou un groupe méthyle et $R^6$ est un groupe méthyle.

5. Une composition selon l'une quelconque des revendications précédentes, dans laquelle Y représente un atome de soufre.

6. Un composé de la formule générale I ou, quand c'est possible, un sel d'addition d'acide d'un tel composé comme défini dans la revendication 1, où Y est du soufre, avec la condition qu'un des substituants $R^1$ et $R^2$ ne peut pas être un groupe méthyle quand l'autre est un groupe éthoxycarbonyle et avec la condition en outre que quand $R^1$ et $R^2$ représentent ensemble une liaison alcoylène de 3 ou 4 atomes de carbone, la chaîne carbonée de la liaison doit être substituée par au moins un des groupes indiqués comme substituants éventuels et quand la liaison alcoylène contient 3 atomes de carbone, l'atome de carbone du milieu ne doit pas être substitué par deux groupes méthyle si les deux autres atomes de carbone sont non-substitués.

7. Un composé de formule II ou, quand c'est possible, un sel d'addition d'acide d'un tel composé, comme défini dans les revendications 2 à 4, avec la condition que Z et $Z^1$ ne peuvent pas être tous deux simultanément de l'hydrogène quand Y représente un groupe

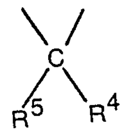

et $R^4$ et $R^5$ sont identiques, représentant chacun de l'hydrogène ou un groupe méthyle.

8. Un procédé pour la préparation d'un composé tel que revendiqué dans la revendication 6, qui comprend la réaction d'une dioxime de la formule générale:

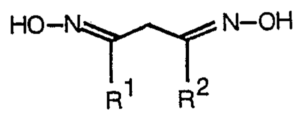

(III)

avec du monochlorure de soufre et/ou du dichlorure de soufre pour donner un produit de réaction contenant au moins un composé de formule I dans lequel X est de l'oxygène ou du soufre et éventuellement un second composé de formule I qui diffère du premier composé de formule I seulement en ce qui concerne la nature du substituant X, X représentant de l'oxygène dans un composé et X représentant du soufre dans l'autre composé et, si les deux composés de formule I sont présents dans le produit de réaction, la séparation éventuelle des deux composés du produit de réaction sous la forme de constituants individuels.

9. Un procédé de lutte contre la croissance de plantes indésirables en un lieu, qui comprend le traitement du lieu par un composé selon la revendication 6 ou 7 ou une composition selon l'une quelconque des revendications 1 à 5.

**Revendications pour l'Etat contractant: AT**

1. Une composition herbicide qui comprend au moins deux véhicules, dont l'un au moins est un agent tensio-actif, en même temps que, comme ingrédient actif, un composé de la formule générale I ou, quand c'est possible, un sel d'addition d'acide d'un tel composé:

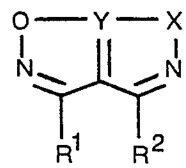

(I)

où Y représente un atome de soufre, de sélénium ou de tellure; $R^1$ et $R^2$ représentent chacun indépendamment un groupe alcoyle ayant jusqu'à 6 atomes de carbone, un groupe phényle ou un groupe

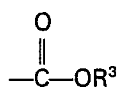

où $R^3$ est un groupe alcoyle ayant jusqu'à 6 atomes de carbone; ou $R^1$ et $R^2$ représentent ensemble une liaison alcoylène contenant jusqu'à 4 atomes de carbone dans la chaîne ou une liaison alcoylène contenant jusqu'à 3 atomes de carbone et un hétéro-atome choisi parmi le soufre, l'azote et l'oxygène dans la chaîne, chaque liaison étant éventuellement substituée par jusqu'à 3 groupes alcoyle contenant chacun jusqu'à 12 atomes de carbone, jusqu'à 3 groupes phényle, un groupe benzyle, un groupe acyle ayant jusqu'à 12 atomes de carbone, un groupe pyridyle ou un sel correspondant, un groupe phényle alcoxylé contenant jusqu'à 6 atomes de carbone dans la portion alcoyle de la portion alcoxy ou un groupe furyle et, quand l'hétéro-atome est du soufre, ses dérivés oxydés; et X représente un atome d'oxygène ou, quand Y représente un atome de soufre, X peut en variante représenter un atome de soufre.

2. Une composition selon la revendication 1 dans laquelle l'ingrédient actif est un composé de la formule II ou, quand c'est possible, un sel d'addition d'acide d'un tel composé:

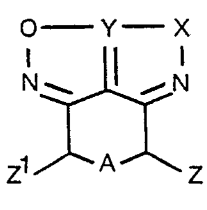

(II)

où X et Y ont les significations indiquées dans la revendication 1, Z représente un atome d'hydrogène, un groupe alcoyle ayant jusqu'à 12 atomes de carbone, un groupe phényle ou un groupe pyridyle ou un sel correspondant ou un groupe benzyle; $Z^1$ représente un atome d'hydrogène, un groupe alcoyle ayant jusqu'à 6 atomes de carbone, un groupe pyridyle ou un sel correspondant ou un groupe phényle et A représente un groupe:

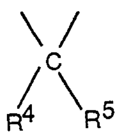 , 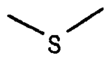 , 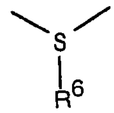 , 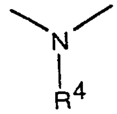 , 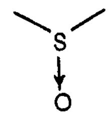 , 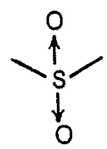 ou 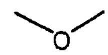

où $R^4$ représente un atome d'hydrogène, un groupe alcoyle ayant jusqu'à 12 atomes de carbone, un groupe benzyle ou un groupe pyridyle ou un sel correspondant, ou un groupe

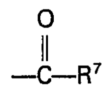

où $R^7$ est un groupe alcoyle ou alcényle ayant jusqu'à 12 atomes de carbone, un groupe aryle ayant jusqu'à 12 atomes de carbone, un groupe aralcoyle ayant jusqu'à 9 atomes de carbone ou un groupe alcaryle ayant jusqu'à 9 atomes de carbone, $R^5$ représente un atome d'hydrogène ou un groupe alcoyle ayant jusqu'à 6 atomes de carbone et $R^6$ représente un groupe alcoyle ayant jusqu'à 12 atomes de carbone ou un groupe benzyle, avec la condition que Z et $Z^1$ ne peuvent pas être tous deux simultanément un groupe pyridyle ou un sel correspondant.

3. Une composition selon la revendication 2, dans laquelle Z et $Z^1$ représentent chacun un atome d'hydrogène ou un groupe alcoyle ayant jusqu'à 3 atomes de carbone et A est un groupe:

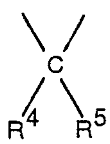 , 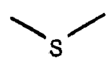 ou 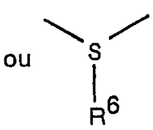 .

où $R^4$ est un atome d'hydrogène ou un groupe alcoyle ayant jusqu'à 9 atomes de carbone, $R^5$ est un atome d'hydrogène ou un groupe alcoyle ayant jusqu'à 3 atomes de carbone et $R^6$ est un groupe alcoyle ayant jusqu'à 9 atomes de carbone.

4. Une composition selon la revendication 3, dans laquelle $R^4$ et $R^5$ représentent chacun un atome d'hydrogène ou un groupe méthyle et $R^6$ est un groupe méthyle.

5. Une composition selon l'une quelconque des revendications précédentes, dans laquelle Y représente un atome de soufre.

6. Un procédé pour la préparation d'un composé de formule I tel que défini par l'une quelconque des revendications 1 à 6, qui comprend la réaction d'une dioxime de la formule générale:

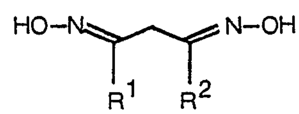

(III)

avec du monochlorure de soufre et/ou du dichlorure de soufre pour donner un produit de réaction contenant au moins un composé de formule I dans lequel X est de l'oxygène ou du soufre et éventuellement un second composé de formule I qui diffère du premier composé de formule I seulement en ce qui concerne la nature du substituant X, X représentant de l'oxygène dans un composé et X représentant du soufre dans l'autre composé et, si les deux composés de formule I sont présents dans le produit de réaction, la séparation éventuelle des deux composés du produit de réaction sous la forme de constituants individuels.

7. Un procédé de lutte contre la croissance de plantes indésirables en un lieu, qui comprend le traitement du lieu par un composé ou une composition selon l'une quelconque des revendications 1 à 5.